(19) Europäisches Patentamt
European Patent Office
Office européen des brevets

(11) EP 3 679 349 B1

(12) EUROPÄISCHE PATENTSCHRIFT

(45) Veröffentlichungstag und Bekanntmachung des Hinweises auf die Patenterteilung:
**06.01.2021 Patentblatt 2021/01**

(21) Anmeldenummer: **18745937.5**

(22) Anmeldetag: **25.07.2018**

(51) Int Cl.:
***G01N 21/3577*** *(2014.01)* ***G01N 33/28*** *(2006.01)*
***G01N 21/35*** *(2014.01)*

(86) Internationale Anmeldenummer:
**PCT/EP2018/070229**

(87) Internationale Veröffentlichungsnummer:
**WO 2019/048124 (14.03.2019 Gazette 2019/11)**

# (54) VERFAHREN ZUR QUANTITATIVEN BESTIMMUNG VON FETTSÄUREESTERN IN TREIBSTOFFEN

METHOD FOR THE QUANTITATIVE DETECTION OF FATTY ACID ESTERS IN FUELS

MÉTHODE DE DÉTERMINATION QUANTITATIVE D'ESTERS D'ACIDE GRAS DANS DES CARBURANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.09.2017 EP 17189615**

(43) Veröffentlichungstag der Anmeldung:
**15.07.2020 Patentblatt 2020/29**

(73) Patentinhaber: **Quantared Technologies GmbH
1100 Wien (AT)**

(72) Erfinder:
- **RITTER, Wolfgang
1100 Wien (AT)**
- **LENDL, Bernhard
1100 Wien (AT)**

(74) Vertreter: **Sonn & Partner Patentanwälte
Riemergasse 14
1010 Wien (AT)**

(56) Entgegenhaltungen:
- **MUHAMMAD TARIQ ET AL: "Activity of homogeneous and heterogeneous catalysts, spectroscopic and chromatographic characterization of biodiesel: A review", RENEWABLE AND SUSTAINABLE ENERGY REVIEWS., Bd. 16, Nr. 8, 30. August 2012 (2012-08-30), Seiten 6303-6316, XP055446618, US ISSN: 1364-0321, DOI: 10.1016/j.rser.2012.07.005**
- **Gerhard Knothe: "Monitoring a Progressing Transesterification Reaction by Fiber-Optic Near Infrared Spectroscopy with Correlation to 1H Nuclear Magnetic Resonance Spectroscopy", , 2000, XP055446573, Gefunden im Internet: URL:http://lib3.dss.go.th/fulltext/Journal/J.AOCS/J.AOCS/2000/no.5/may2000vol77,no5, p489-493.pdf [gefunden am 2018-01-31]**
- **ALCARÁZ MIRTA R ET AL: "EC-QCL mid-IR transmission spectroscopy for monitoring dynamic changes of protein secondary structure in aqueous solution on the example of beta-aggregation in alcohol-denaturated alpha-chymotrypsin", ANALYTICAL AND BIOANALYTICAL CHEMISTRY, SPRINGER, DE, Bd. 408, Nr. 15, 23. März 2016 (2016-03-23), Seiten 3933-3941, XP035867778, ISSN: 1618-2642, DOI: 10.1007/S00216-016-9464-5 [gefunden am 2016-03-23]**
- **GREG HEMIGHAUS ET AL: "Alternative Jet Fuels", A SUPPLEMENT TO CHEVRON'S AVIATION FUELS TECHNICAL REVIEW, CHEVRON CORPORATION , 2006, Seiten 1-13, XP002620000, Gefunden im Internet: URL:http://www.chevronglobalaviation.com/docs/5719_Aviation_Addendum._webpdf.pdf [gefunden am 2011-02-01]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 679 349 B1

## Beschreibung

Die vorliegende Erfindung betrifft ein Verfahren zur quantitativen Bestimmung von Verunreinigungen in Form von Analyten basierend auf Ester, insbesondere Fettsäureester, wie z.B. Fettsäuremethyl- bzw. ethylester (FAME/FAEE) beziehungsweise Glyceriden in Flugturbinentreibstoffen (Jet Fuel).

Rasche und genaue Routinemessungen von Verunreinigungen, Additiven oder Bestandteilen von flüssigen Kraftstoffen zählen zu den Hauptaufgaben der industriellen Messtechnik. Kraftstoffe werden nach strengen Spezifikationen hergestellt, können aber während dem Transport in Pipelines, Tankern, Lagertanks oder Filtern verunreinigt werden. Zusätzlich ist es erlaubt Additive an verschiedenen Punkten der Lieferkette beizugeben.

Als Fettsäuremethylester bzw. fatty acid methyl ester (FAME) werden die Ester einer Fettsäure mit Methanol als Alkohol bezeichnet, entsprechend heißen die Ester einer Fettsäure mit Ethanol als Alkohol Fettsäureethylester bzw. fatty acid ethyl ester (FAEE). Gemische aus FAMEs und FAEEs, gewonnen aus pflanzlichen oder tierischen Fetten (z. B. Rapsöl), werden als Kraftstoff für Dieselmotoren genutzt und im Allgemeinen als Biodiesel bezeichnet. In vielen Ländern ist die Beimischung von Biodiesel zu Dieselkraftstoff bereits gesetzlich vorgeschrieben. Zusätzlich zu FAME und FAEE können auch Spuren deren Ausgangsprodukte (Glyceride) im Kraftstoff enthalten sein.

FAME, FAEE und Glyceride sind bei Raumtemperatur fest oder flüssig und kommen mit einem Teil ihrer Eigenschaften denen von Dieselkraftstoff sehr nahe, sind jedoch zugleich Lösungsmittel mit anderen Wirkungen als Dieselkraftstoff, was zu technischen Problemen an Dichtungsmaterialien in Motorensystemen führen kann. Fettsäuremethylester und -ethylester können daher grundsätzlich nur dann als alternativer Kraftstoff verwendet werden, wenn die Dichtungsmaterialien im verwendeten Motor gegen FAME bzw. FAEE beständig sind.

In den heutzutage verwendeten weltweiten Versorgungssystemen, wie Pipelines, Tanklastwägen, Zügen und Schiffen, werden oft die verschiedensten Treibstoffe und Flüssigkeiten nacheinander transportiert, diese jedoch unter Verwendung von Schutzmaßnahmen, die dafür ausgelegt sind um Kreuz-Kontaminierung zu minimieren. Speziell FAME hat jedoch die Eigenschaft, fest an Oberflächen zu haften, wodurch kleine Mengen an FAME auch in Flugturbinentreibstoffen (Jet Fuel) gefunden werden können. Diese Verunreinigungen rührt zumeist davon, dass Jet Fuel nach FAME und/oder FAEE enthaltenden Treibstoffen im gleichen Transportsystem geliefert wurden. Zusätzlich können FAME und FAEE aus Biodiesel in Multiprodukt-Pipelines an den Wänden haften bleiben und somit nachfolgendes Jet Fuel verunreinigen.

Von der Joint Inspection Group (JIG) wurden FAME in Jet Fuel als gefährliche Substanzen eingestuft. Studien zeigen bereits bei geringer Konzentration potentielle Gefahren. Diese reichen von der Korrosion von Lagern, Ablagerungen in der Treibstoffdüse, Beeinträchtigungen von Dichtmaterialen bis zu Veränderung der Viskosität des Treibstoffes bei geringen Temperaturen in großer Höhe. Die internationalen Spezifikationen Def.Stan. 91.-91 und ASTM D1655 schreiben zurzeit ein Limit von < 50 mg/kg FAME in Jet Fuel vor. Bei höheren Werten wird ein Betanken der Flugzeuge und Starten der Triebwerke verboten bzw. sind bei irrtümlichem Betrieb von Triebwerken mit Jet Fuel mit mehr als 50 mg/kg FAME zwingend zusätzliche Wartungsarbeiten für die Triebwerke vorgeschrieben.

Zur Bestimmung des FAME/FAEE Gehalts in Jet Fuel werden zum gegenwärtigen Zeitpunkt im Wesentlichen vier Verfahren eingesetzt. Dabei handelt es sich um drei chromatographische Verfahren, die Gaschromatographie-Massenspektroskopie (GC-MS), die Hochleistungsflüssigkeitschromatographie (HPLC) und die Heartcut-Gaschromatographie; sowie ein Fourier-Transformations-Infrarot-Spektrometrie (FTIR) Verfahren. Zur quantitativen Konzentrationsbestimmung von FAME/FAEE/Glyceride wird zurzeit vor allem GC-MS eingesetzt. Dieses Verfahren ist zwar genau und spezifisch, kann jedoch nur in einem qualifizierten Analysenlabor durchgeführt werden und hat für die Analyse einen hohen Zeitbedarf. Zusätzlich können mit chromatographischen Verfahren kurzkettige FAME, z.B. Kokosnuss Methyl Ester, nicht von anderen Bestandteilen des Jet Fuels unterschieden werden.

Bei der Herstellung von Biodiesel wird der Umesterungsprozess von pflanzlichen Fetten und Ölen überwacht, indem der Reaktionsumsatz mittels FTIR-Messung analysiert wird. Tariq et al. beschreibt in Renewable and Sustainable Energy Reviews, Bd. 16, Nr. 8, die Herstellung von Biodiesel, der sich aus Fettsäuremethylestern zusammensetzt, durch Umesterung verschiedener pflanzlicher Öle. Dabei wird beispielsweise der Anteil von Fettsäuren im Gemisch der Reaktionsprodukte mittels FTIR-Spektroskopie ermittelt. Knothe beschreibt im Journal of the American Oil Chemists' Society, Vol. 77, Nr. 5, bei der Herstellung von Biodiesel die Überwachung des Umesterungsprozesses eines pflanzlichen Öls zu Fettsäuremethyleser mittels IR-Spektroskopie. Die Verwendung von IR-Spektroskopie, die auf einem Quantenkaskadenlaser basiert, wird von Alcaräz et al. im Journal Analytival and Bioanalytical Chemistry bei der Überwachung dynamischer Änderungen in Sekundärstrukturen von Proteinen in wässrigen Lösungen beschrieben.

Es besteht also der dringende Bedarf an einem vereinfachten Analyseverfahren für FAME, um idealerweise Vor-Ort- oder in einem Feldlabor Analysen durchführen zu können.

In den letzten Jahren wurde ein FTIR Analysenverfahren entwickelt, bei welchem die zu analysieren Probe (Jet Fuel + FAME) durch eine Einweg-Kartusche geleitet wird, welche mit einen Sorbensmaterial (beispielsweise Silikagel) gefüllt ist. Durch dieses Sorbensmaterial werden FAME mittels Adsorption innerhalb der Kartusche festgehalten, wodurch die hindurchgeleitete Probe danach frei von FAME ist. Mittels einer FTIR-Messung wird die Probe vor und nach

der Kartusche verglichen, um damit den FAME-Gehalt zu bestimmen. Dafür wird vor allem die Bande der Carbonylgruppe um 1749 $cm^{-1}$ ausgewertet. Nachteilig daran ist, dass das Sorbensmaterial zumeist eine nicht ausreichende Selektivität für FAME zeigt, sodass auch andere (polare) Substanzen adsorbiert werden. Daher ist bei diesem Analyseverfahren zusätzlich ein chemometrisches Modell (PLS) notwendig, um den Einfluss dieser Störsubstanzen herauszurechnen.

K Oden et al. offenbaren in "Analysis of Biodiesel Fatty Acid Methyl Esters (FAME) in Aviation Jet Fuels Using Method IP 585" (2016) ein Gaschromatographie-Massenspektroskopie-Verfahren zur Messung von Verunreinigungen in Flugturbinentreibstoffen durch FAME. Das Verfahren bedient sich für die Messung von Biodiesel in Flugturbinentreibstoff der unterschiedlichen Polarität von FAME und Flugturbinentreibstoff. Bei diesen Methoden finden keine chemischen Reaktionen statt.

Aufgabe der vorliegenden Erfindung ist es daher, ein Messverfahren für Ester, insbesondere Fettsäureester (FAME/FAEE aber auch Glyceriden) in Flugturbinentreibstoffen (Jet Fuel) zur Verfügung zu stellen, welches Messverfahren die Nachteile des Standes der Technik nicht aufweist. Die Aufgabe wird durch ein Verfahren gemäß Anspruch 1 gelöst. Erfindungsgemäß wird zur quantitativen Bestimmung von Verunreinigungen in Form von Fettsäureester, wie Fettsäuremethyl- bzw. etylester und/oder Glyceriden, in Flugturbinentreibstoffen der Analyt (z.B. FAME/FAEE/Glyceride) einer für ihn selektiven, die Intensität der Carbonylbande der jeweiligen Estergruppe beeinflussenden chemischen Reaktion unter Bildung eines modifizierten Analyten unterzogen und die Änderung der Konzentration an Analyt in der Probe unter Heranziehung der Abnahme der Intensität der Carbonylbande und/oder die Zunahme der Konzentration des modifizierten Analyten unter Heranziehung der Zunahme der Intensität einer für die Modifizierung charakteristischen Bande gemessen. Durch die gegenüber der im Stand der Technik verwendeten Adsorption höhere Selektivität der selektiven chemischen Reaktion auf den Analyten kann eine höhere Selektivität des Messverfahrens erreicht werden. Durch den Vergleich mit der ursprünglichen, nicht reagierten Probe (enthaltend den Analyten) als spezifischen Hintergrund lassen sich auch Matrix-Effekte korrigieren. Deutlich weniger Störstoffe gehen diese selektive chemische Reaktion ein und damit wird das Verfahren für verschiedenste Proben robuster und weniger störempfindlich. Die Nachweisgrenze kann gesenkt werden, die höhere Selektivität der "Probenvorbereitung" erlaubt es, mit einzelnen Wellenlängenpunkten im Spektrum eine robuste Auswertung zu erzielen. Damit gelingt es in einer bevorzugten Ausführungsform des erfindungsgemäßen Verfahrens, ein spektral weit durchstimmbares FTIR durch Lichtquellen, wie zum Beispiel Quantenkaskadenlaser (QCL), mit limitierter spektraler Durchstimmbarkeit zu ersetzen. Vorteilhaft an dem erfindungsgemäßen Verfahren sind:

- höhere Selektivität
- höhere Robustheit gegenüber Störstoffen in verschiedenen Jet Fuel
- verbesserte Nachweisstärke
- Miniaturisierung durch QCL
- reduzierte Herstellkosten

Gemäß einer bevorzugten Ausführungsform der vorliegenden Erfindung erfolgt die Messung der Änderung der Konzentration an Analyt durch IR-Spektroskopie.

Günstig ist dabei, wenn die Messung der Änderung der Konzentration an Analyt durch FTIR-Spektroskopie erfolgt.

Auch hat sich als günstig herausgestellt, wenn die Messung der Änderung der Konzentration an Analyt durch IR-Spektroskopie unter Verwendung von Quantenkaskadenlaser erfolgt. Insbesondere durch Verwendung der QCL ist es möglich, die Änderung der Intensität einer Bande bei Analyten oder modifizierten Analyten in einem kleinen spektralen Fenster auswerten zu können

Jet Fuel ist kein Reinstoff, sondern ein Gemisch aus Paraffinen, Naphthenen und Aromaten in verschiedenen Verhältnissen. Durch die Zugabe von Additiven wird je nach Zusammensetzung garantiert, dass das Gemisch die notwendigen Spezifikationen erfüllt. Daraus folgt, dass Jet Fuel aus verschiedenen Raffinerien, Pipelines oder sogar Batches aus derselben Quelle spektral gesehen sehr unterschiedlich sein kann. Deshalb ist es im Allgemeinen mit optischen Messmethoden nicht möglich, geringe Konzentrationen von Bestandteilen in Jet Fuel ohne Generation eines probenspezifischen Hintergrunds zu messen.

Beim erfindungsgemäßen Verfahren wird die Probe (Jet Fuel + FAME/FAEE/Glycerid) im Zuge der Messung einer selektiven Reaktion unterzogen, so wird beispielsweise gemäß einer bevorzugten Ausführungsform der vorliegenden Erfindung FAME/FAEE/Glycerid oder allgemein ein zu bestimmender Ester bzw. Fettsäureester durch Zugabe eines Amins in das entsprechende Amid umgewandelt, wobei die Änderung der Konzentration an FAME/FAEE/Glycerid bzw. allgemein die Änderung der Konzentration an zu bestimmenden Ester bzw. Fettsäureester in der Probe anhand der

Abnahme der Intensität der Carbonylbande des Esters und/oder der Zunahme der Intensität der Amidbande des gebildeten Amids gemessen werden kann.

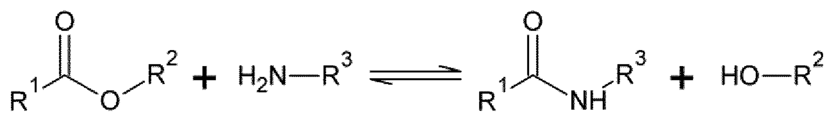

*Aminolyse eines Esters.*

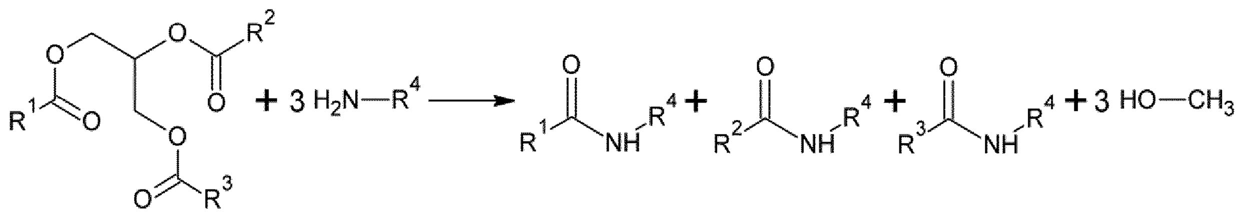

*Aminolyse eines Triglycerids.*

Gemäß einer weiteren bevorzugten Ausführungsform des erfindungsgemäßen Verfahrens wird der Ester bzw. FAME/FAEE/Glycerid durch Zugabe eines anderen Alkohols unter geeigneten Reaktionsbedingungen in den entsprechenden anderen Ester umgewandelt, wobei die Änderung der Konzentration an FAME/FAEE/Glycerid bzw. allgemein die Änderung der Konzentration an zu bestimmenden Ester bzw. Fettsäureester in der Probe anhand der Abnahme der Intensität der Carbonylbande des Esters und/oder der Zunahme der Intensität der Carbonylbande des anderen Esters gemessen wird. Die selektive Reaktion ist dabei eine Umesterung der Fettsäureester durch Zugabe eines anderen Alkohols. Der gebildete Ester (d. h. der modifizierte Analyt) zeigt eine verschobene Bande der Carbonylgruppe, welche von der ursprünglichen Bande unterschieden werden kann. Ausgewertet wird wiederum die Abnahme der Carbonylbande des Analyts Ester, FAME/FAEE bzw. des Glycerids und/oder die Zunahme der Carbonylbande des modifizierten Analyts. Leicht nachteilig kann bei diesem Verfahren der Umesterung eine spektrale Nähe der Produktbande zur Messwellenlänge und damit eine mögliche Beeinflussung der Messung sein.

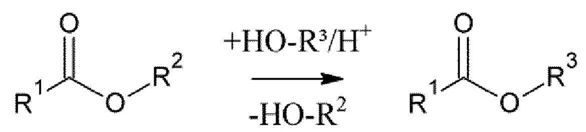


*Umesterung mittels eines Alkohols.*

In beiden dargestellten Reaktionen ändern sich die spektralen Eigenschaften der Probe, wodurch eine Bestimmung des Gehalts des Analyten mittels Vergleichsmessungen möglich wird. Dabei kann sowohl die Abnahme der Intensität der Carbonylbande des Analyten als auch die Zunahme der Intensität einer spezifischen Bande im modifizierten Analyten für das erfindungsgemäße Messverfahren herangezogen werden, wobei bei Berücksichtigung beider Änderungen sowie der Beziehung der Änderungen zueinander die quantitative Bestimmung des Analyten weiter vereinfacht wird.

Die beiliegende Fig. 1 zeigt den Abbau von FAME (1) anhand der Abnahme der Carbonylstreckschwingung um 1749 $cm^{-1}$ und die spektral hinreichend verschobene Zunahme des entsprechenden Amids als Reaktionsprodukt (2) im Infrarot-Absorptionsspektrum.

Gemäß einer bevorzugten Ausführungsform der vorliegenden Erfindung erfolgt die Reaktion zur Bildung des modifizierten Analyten enzymatisch katalysiert, besonders bevorzugt in Anwesenheit von Lipase. Auch die Verwendung anderer Enzyme ist denkbar, wobei diese Enzyme, bzw. Katalysatoren im Allgemeinen, die Reaktion zur Modifizierung des Analyten möglichst vollständig ablaufen lassen sollen.

Durch die selektive chemische Reaktion wird die Konzentration an FAME/FAEE/Glyceriden in der Probe verringert, dadurch kommt es im Fall von FAME im Infrarot-Spektrum zu einer Abnahme/Verschiebung der Carbonylbande (1749 $cm^{-1}$) bzw. im Fall der Aminolyse zu einem Hinzutreten einer Amid-Bande (1690 $cm^{-1}$). Die Abnahme der ursprünglichen Carbonylbande wird vermessen und über eine Kalibration in die gesuchte Analyt-Konzentration umgerechnet. Eine weitere mögliche Ausführungsform ist die Bestimmung der Konzentration des Analyten über die Messung der Zunahme der Intensität der Amidbande.

Die Carbonylgruppe der FAEE zeigt ihr Absorptionsmaxima bei 1742 $cm^{-1}$, gemäß einer bevorzugten Ausführungsform der vorliegenden Erfindung kann beispielsweise durch symmetrische Auswertung um 1742 $cm^{-1}$ die Konzentration an FAEE bestimmt bzw. herausgerechnet werden. Unter symmetrischer Auswertung wird dabei insbesondere eine Auswertung bei +/- 2 $cm^{-1}$, insbesondere +/- 5 $cm^{-1}$, besonders bevorzugt +/- 10 $cm^{-1}$ um die genannten Absorptionsmaxima verstanden, selbstverständlich kann die Bestimmung der Konzentration an Analyten aber auch gemäß anderen, dem Fachmann bekannten Verfahren erfolgen.

Eine mögliche unerwünschte Nebenreaktion bei Verwendung der Aminolyse im erfindungsgemäßen Verfahren ist die Umwandlung von FAME/FAEE/Glyceride mittels Alkoholen in andere Ester. Diese Ester zeigen ihr Absorptionsmaxima bei ca. 1742 $cm^{-1}$, welches eine Messung bei 1749 $cm^{-1}$ stören kann. Durch die oben erwähnte symmetrische Auswertung um 1742 $cm^{-1}$ kann dieser Effekt ebenfalls herausgerechnet bzw. minimiert werden.

Gemäß einer bevorzugten Ausführungsform des erfindungsgemäßen Verfahrens weist das Verfahren zusätzlich zur quantitativen Bestimmung unter Heranziehung der/einer Reaktionskinetik eine qualitative Bestimmung auf. Ein Vorteil dieses Verfahrens ist, dass durch diese qualitative Bestimmung Fettsäureester von anderen Analyten, wie beispielsweise anderen Ketonen, unterschieden werden können und somit ein Verfälschen der quantitativen Bestimmung der Verunreinigungen in Form von Fettsäureestern in Flugturbinentreibstoffen durch andere Analyten minimiert.

Gemäß einer bevorzugten Ausführungsform des erfindungsgemäßen Verfahrens wird das Verfahren zur Identifizierung bzw. zum Ausschluss der Identität der Verunreinigung verwendet. Mit dem erfindungsgemäßen Verfahren kann im Ausschlussverfahren bestimmt werden, um welche chemischen Verbindungen es sich bei den Verunreinigungen im Flugturbinentreibstoff handelt. Zwar kann die Identität der Verunreinigung selbst nicht mit letzter Genauigkeit bestimmen werden, es können aber gewisse Stoffe ausgeschlossen werden. Beispielsweise schließt eine stark verlangsamte Reaktion beim erfindungsgemäßen Verfahrens aus, dass es sich bei der aufgefundenen Verunreinigung im Flugturbinentreibstoff um FAEE/FAME/Triglyceride handelt.

In den beiliegenden Figuren zeigen:

Fig. 1: Abbau von FAME (1) anhand der Abnahme der CarbonylStreckschwingung um 1749 cm-1 und der spektral hinreichend verschobenen Zunahme des entsprechenden Amids als Reaktionsprodukt (2) im Infrarot Absorptionsspektrum.

Fig. 2: Kinetik der enzymatisch-katalytischen Reaktion von FAME und Amin zum entsprechenden Amid (Punkte) mit exponentielle Fit (Linie) zur Messwertvorhersage.

Fig. 3: Vergleich der Reaktionskinetik der Umesterung und der Aminolyse.

Fig. 4: Umsetzungen von Fettsäurestern und anderen Analyten, gemessen an einem Messsystem mit FTIR gemäß dem erfindungsgemäßen Verfahren.

Die vorliegende Erfindung wird im Folgenden anhand der genannten Beispiele erläutert.

Folgende Bestimmungen wurden durchgeführt:

- FAME in Jet Fuel mittels Aminolyse
- FAEE in Jet Fuel mittels Aminolyse
- Glyceryl trioleate in Jet Fuel mittels Aminolyse
- FAME in Diesel mittels Aminolyse
- FAME in Jet Fuel durch Umesterung

Für die Bestimmung von FAME in Jet Fuel mittels Aminolyse wurden zusätzliche Messungen vorgenommen:

- zugelassene Jet Fuel Additive
- Jet Fuel Screening.

Allgemeiner Methodenablauf

Messgeräte zur Durchführung der Methode bestehen in einer bevorzugten Ausführung aus einer Flusszelle, einer Infrarotquelle (z.B.: Globar mit oder ohne spektralem Filter oder QCL) samt Detektor, Pumpen, Ventilen, einem System zur Zudosierung des Reaktanten sowie einer Mehrweg-Kartusche die den Katalysator enthält. Die Schichtdicke der Flusszelle wird typischerweise auf die Lichtleistung der verwendeten Infrarotquelle und die IR Transmission der Probenmatrix abgestimmt.

Am Beginn einer Messsequenz wird das Messgerät mit der zur analysierenden Probenflüssigkeit gespült, danach wird ein Hintergrund mit QCL oder FTIR aufgenommen. Nach Zugabe eines der nachstehend angeführten Reaktanden wird die Probenflüssigkeit zwischen Flusszelle und einer den Katalysator enthaltenden Kartusche im Kreis gepumpt. Dabei wird der Analyt einer für ihn selektiven, die Intensität der Carbonylbande der jeweiligen Estergruppe

beeinflussenden chemischen Reaktion unter Bildung eines modifizierten Analyten unterzogen und die Änderung der Konzentration an Analyt in der Probe unter Heranziehung der Abnahme der Intensität der Carbonylbande und/oder die Zunahme der Konzentration des Bildung des modifizierten Analyten unter Heranziehung der Zunahme der Intensität einer für die Modifizierung charakteristischen Bande gemessen. Über die Kinetik der Reaktion lassen sich frühe Schwellwertwarnungen abgegeben, der vollständige Ablauf der Reaktion überprüfen, sowie durch Vermessung von Standardproben die verbleibende Aktivität des Katalysators in der Mehrweg-Kartusche bestimmen. Nach Ablauf der Reaktion wird das Messgerät wieder mit Probenflüssigkeit gespült um dann einen zweiten Hintergrund zu messen. Aus der Änderung der gemessenen Intensität zwischen Hintergrund ($I_0$) und abreagierter Probe ($I$) wird mittels dem Lambert-Beer'schen Gesetz

$$Abs = \log (I_0/I)$$

die Absorption (Abs) bestimmt. Daraus ergibt sich, im Vergleich mit Kalibrationskurven, über

$$\text{Konzentration Analyt [mg/kg]} = (Abs * k + d) * \rho_C/\rho_S$$

die Konzentration des Analyten, wobei

$k$ = Steigung der Kalibrationsgerade
$d$ = Achsenabschnitt der Kalibrationsgerade
$\rho_C$ = Dichte der Kalibrationsmatrix in $kg/m^3$
$\rho_S$ = Dichte der Probe in $kg/m^3$

gilt.

Als Beispiel ergibt sich für eine Messung von Jet Fuel, das mit 49,9 mg/kg FAME versetzt wurde, bei einer gemessenen Absorption von 17,81 mAU mit der Kalibrationsgeraden k = 2,79 mAU-1 und d = -0,18 mg/kg eine berechnete FAME Konzentration von 49,51 mg/kg. Die Dichte der Kalibrationsmatrix und der Probe sind in diesem Beispiel identisch.

FAME in Jet Fuel mittels Aminolyse:

Die Aminolyse hat sich als vorteilhafte Variante herausgestellt, da sie unter enzymatisch-katalytischen Bedingungen für FAME Konzentrationen unter 500 mg/kg in unter 20 Minuten abgeschlossen ist (siehe beiliegende Fig. 2).

***Tabelle 1: FAME in Jet Fuel mittels Aminolyse.***

| Matrix | Analyt | Beigemengte Konzentration [mg/kg] | Berechnete Konzentration [mg/kg] |
|---|---|---|---|
| Jet Fuel | FAME Mix aus B7 | 0,0 | -0,02 |
| Jet Fuel | FAME Mix aus B7 | 5,5 | 5,34 |
| Jet Fuel | FAME Mix aus B7 | 11,1 | 11,26 |
| Jet Fuel | FAME Mix aus B7 | 22,1 | 21,66 |
| Jet Fuel | FAME Mix aus B7 | 199,3 | 200,19 |
| Jet Fuel | FAME Mix aus B7 | 400,4 | 399,98 |

Tabelle 1 zeigt exemplarische Resultate von Jet Fuels, die mit einem FAME-Mix (20 % RME Rape-Methyl-Ester, PME Palm-Methyl-Ester, TME Tallow-Methyl-Ester, SME Soy-Methyl-Ester und UCOME Used-Cooking-Oil-Methyl-Ester als siebenprozentige Lösung in Diesel; B7) in verschiedenen Konzentrationen versetzt wurden.

Die Resultate zeigen, dass FAME in Jet Fuel im Bereich 0 - 250 mg/kg mit einer Genauigkeit von < 1 mg/kg gemessen werden kann. Zur Bestimmung von FAME in Jet Fuel ohne den Einfluss von FAEE und Alkoholen wurden alle Daten symmetrisch um 1742 $cm^{-1}$ ausgewertet.

Das erfindungsgemäße Verfahren wurde nicht nur mit dem zuvor genannten, auf den europäischen Markt abgestimmten FAME Mix getestet, sondern auch mit einzelnen FAME Typen. Die Messergebnisse mit Konzentration um das erlaubte Maximum von 50 mg/kg sind in der untenstehenden Tabelle 2 aufgelistet.

Aus Tabelle 2 wird ersichtlich, dass die Genauigkeit auf einzelne FAME etwas geringer ist, da sich FAME aus

verschiedenen Quellen typischerweise in der Kettenlänge unterscheiden. Bei Short Chain Methyl Estern wie CME (Coconut-Methyl-Ester, wird vor allem in Südostasien verwendet und ist deshalb nicht Teil des auf Europa ausgelegten FAME Mixes) mit kürzeren Kohlenstoffkettenlängen und damit geringerem Molgewicht führt die Beigabe einer Konzentration in mg/kg zu einer größeren Menge an Carbonylgruppen und damit zu einer bekannten systematischen Überbestimmung. Diese Abweichungen lassen sich durch eine gezielte Kalibration leicht und einfach korrigieren. Die zurzeit zugelassenen Methoden können CME entweder nicht bestimmen (GC-MS, GC-Heart Cut und HPCL), oder haben dieselbe systematische Überbestimmung (FTIR Rapid Screening Method).

***Tabelle 2: FAME einzeln im Vergleich zur FAME Mix aus B7 Kalibration.***

| Matrix | Analyt | Beigemengte Konzentration [mg/kg] | Berechnete Konzentration [mg/kg] |
|---|---|---|---|
| Jet Fuel | RME aus B7 | 48,5 | 54,77 |
| Jet Fuel | PME aus B7 | 48,4 | 49,44 |
| Jet Fuel | TME aus B7 | 47,7 | 48,48 |
| Jet Fuel | SME aus B7 | 49,0 | 46,86 |
| Jet Fuel | UCOME aus B7 | 48,7 | 47,37 |
| Jet Fuel | CME aus B7 | 48,1 | 66,50 |

***Additive*** *:*

Bei der maximalen in Jet Fuel erlaubten Konzentration von 15 zugelassenen Additiven verschiedener Typen (Static Dissipator, Lubricity Improver, Fuel System Icing Inhibitor, Metal Deactivator, Antioxidant & Cetane Improver, Biocide) wurde eine Messwertabweichung von < +/- 5 mg/kg gemessen. Dies liegt im Rahmen der erwarteten Methodenreproduzierbarkeit.

***Jet Fuel Screening:***

Die Bestimmung von FAME in 26 unterschiedlichen Jet Fuels zeigte, dass durchaus Abweichungen vom Nominalwert auftreten können (73 % ≤ 5 mg/kg, bzw. 92 % ≤ 7,5 mg/kg). Solche und gravierendere Abweichungen sind aus weltweiten Jet Fuel Erhebungen des britischen Energy Institutes bekannt (n=189; 85% < 5 mg/kg, bzw. 95% < 10 mg/kg, gemessen mit FTIR Rapid Screening Method) und lassen sich auf die unterschiedlichen Zusammensetzungen und damit die spektralen Unterschiede von realen Jet Fuels zurückführen.

<u>FAEE in Jet Fuel mittels Aminolyse</u>

Die Aminolyse funktioniert ebenfalls mit Fatty Acid Ethyl Ester (FAEE), wobei jedoch im Vergleich zu FAME an geänderten Wellenzahlen ausgewertet werden muss. Tabelle 3 zeigt die Resultate einer Kalibration mit FAEE, in diesem Fall Ethylpalmitat, in verschiedenen Konzentrationen bei einer symmetrischen Auswertung um 1749 $cm^{-1}$.

***Tabelle 3: FAEE in Jet Fuel mittels Aminolyse.***

| Matrix | Analyt | Beigemengte Konzentration [mg/kg] | Berechnete Konzentration [mg/kg] |
|---|---|---|---|
| Jet Fuel | FAEE | 0 | 0,89 |
| Jet Fuel | FAEE | 50 | 48,66 |
| Jet Fuel | FAEE | 100 | 100,23 |
| Jet Fuel | FAEE | 200 | 200,22 |

In Proben, welche sowohl FAEE als auch FAME enthalten, lassen sich durch die Wahl der Auswertewellenzahlen die beiden Analyten unabhängig voneinander bestimmen. Tabelle 4 zeigt beispielhaft die Bestimmung von FAME aus einer Probe in der sowohl FAME als auch FAEE enthalten ist.

***Tabelle 4: Bestimmung von FAME in einer FAME & FAEE Mischung.***

| Matrix | Analyt | Beigemengte Konzentration FAME [mg/kg] | Beigemengte Konzentration FAEE [mg/kg] | Berechnete Konzentration FAME [mg/kg] |
|---|---|---|---|---|
| Jet Fuel | FAME & FAEE | 0 | 103,5 | -3,29 |
| Jet Fuel | FAME & FAEE | 198,4 | 103,5 | 196,26 |

Glyceryltrioleat in Jet Fuel mittels Aminolyse

Die Aminolyse von Triglyceriden führt zur Bildung von Amiden und Alkohol. Diese Reaktion wurde mit Glyceryltrioleat in Jet Fuel mit Lipase als Katalysator durchgeführt. Tabelle 5 zeigt die Resultate für verschiedene Konzentrationen.

***Tabelle 5: Triglyceride in Jet Fuel mittels Aminolyse.***

| Matrix | Analyt | Beigemengte Konzentration [mg/kg] | Berechnete Konzentration [mg/kg] |
|---|---|---|---|
| Jet Fuel | Glyceryltrioleat | 0,0 | -0,98 |
| Jet Fuel | Glyceryltrioleat | 5,0 | 3,14 |
| Jet Fuel | Glyceryltrioleat | 19,9 | 20,18 |
| Jet Fuel | Glyceryltrioleat | 49,8 | 48,65 |
| Jet Fuel | Glyceryltrioleat | 98,5 | 99,61 |

FAME in Diesel mittels Aminolyse

Um zu zeigen, dass das erfindungsgemäße Verfahren auch auf andere flüssige Kraftstoffe ausgeweitet werden kann, wurden Spuren von FAME in einer Diesel B0 Matrix vermessen. Die Resultate der enzymatisch-katalytischen Aminolyse von FAME in Diesel sind in Tabelle 6 aufgelistet.

***Tabelle 6: FAME in Diesel mittels Aminolyse.***

| Matrix | Analyt | Beigemengte Konzentration [mg/kg] | Berechnete Konzentration [mg/kg] |
|---|---|---|---|
| Diesel | FAME Mix aus B7 | 0,0 | -0,27 |
| Diesel | FAME Mix aus B7 | 10,0 | 10,27 |
| Diesel | FAME Mix aus B7 | 19,9 | 20,86 |
| Diesel | FAME Mix aus B7 | 49,2 | 50,19 |
| Diesel | FAME Mix aus B7 | 198,7 | 198,08 |

FAME in Jet Fuel durch Umesterung

Die Umesterung von FAME mit Alkoholen führt zur Bildung von anderen Estern. Durch den geringeren Abstand der charakteristischen Banden zwischen Analyt und Reaktionsprodukt im Vergleich zur Aminolyse, ergeben sich bei der Umesterung etwas schlechtere Werte für Methodenreproduzierbarkeit und Messwertabweichung. Aus der in Fig. 3 gezeigten Reaktionskinetik wird ersichtlich, dass die Umesterung ca. zehn Minuten länger dauert als die Aminolyse. Aus diesen Gründen hat sich die katalytisch-enzymatische Aminolyse von FAME in Jet Fuel als bevorzugte Durchführung des Verfahrens durchgesetzt.

Umsetzung von Fettsäureestern und anderen Analyten

Fig. 4 zeigt die Umsetzungen von FAME, FAEE, Triglyceriden, Diethylhexyladipat und 2-Ethylhexylacetat, gemessen mit einem Messsystem mit FTIR gemäß dem erfindungsgemäßen Verfahren. Die Wellenzahlen der Auswertung wurden hierfür auf den jeweiligen Analyten angepasst.

Aus dem Verlauf der Kurven in Fig. 4 ist ersichtlich, dass über die Reaktionskinetik der Analyten eine qualitative Aussage über die Art des Analyten getroffen werden kann. Zum Beispiel haben der Weichmacher Diethylhexyladipat und der Interferent 2-Ethylhexylacetat, verglichen mit FAME, FAEE und Triglyceriden, eine deutlich langsamere Reaktionsgeschwindigkeit. Demzufolge können FAME, FAEE und Triglyceride von den anderen untersuchten Analyten unterschieden werden, die als Interferenten FTIR-Messungen nach dem Stand der Technik die quantitative Bestimmung von FAME, FAEE und Triglyceriden verfälschen können. Durch die Kombination aus optimierten Auswertewellenzahlen und dem Verlauf der Reaktionskinetik der Analyten können weitere Stoffe getrennt voneinander betrachtet werden. Die Methode zur getrennten Messung und Bestimmung von Verunreinigungen durch Fettsäureester lässt sich auch auf andere Stoffe anwenden.

**Patentansprüche**

1. Verfahren zur quantitativen Bestimmung von Verunreinigungen in Form von Fettsäureestern in Flugturbinentreibstoffen, wobei der Analyt der Fettsäureester Fettsäuremethylester (FAME) und/oder der Fettsäureester Fettsäureethylester (FAEE) ist, wobei der Analyt einer für ihn selektiven, die Intensität der Carbonylbande der jeweiligen Estergruppe beeinflussenden chemischen Reaktion unter Bildung eines modifizierten Analyten unterzogen und die Änderung der Konzentration an Analyt in der Probe, die der Flugturbinentreibstoff zusammen mit FAME und/oder FAEE ist, unter Heranziehung der Abnahme der Intensität der Carbonylbande und/oder die Zunahme der Konzentration des modifizierten Analyten unter Heranziehung der Zunahme der Intensität einer für die Modifizierung charakteristischen Bande gemessen wird.

2. Verfahren nach Anspruch 1, **dadurch gekennzeichnet, dass** die Messung der Änderung der Konzentration an Analyt durch IR-Spektroskopie erfolgt.

3. Verfahren nach Anspruch 2, **dadurch gekennzeichnet, dass** die Messung der Änderung der Konzentration an Analyt durch FTIR-Spektroskopie erfolgt.

4. Verfahren nach Anspruch 2, **dadurch gekennzeichnet, dass** die Messung der Änderung der Konzentration an Analyt durch IR-Spektroskopie unter Verwendung von Quantenkaskadenlaser erfolgt.

5. Verfahren nach einem der Ansprüche 1 bis 4, **dadurch gekennzeichnet, dass** FAME/FAEE durch Zugabe eines Amins in das entsprechende Amid umgewandelt wird, wobei die Änderung der Konzentration an FAME/FAEE in der Probe anhand der Abnahme der Intensität der Carbonylbande des Esters und/oder der Zunahme der Intensität der Amidbande des gebildeten Amids gemessen wird.

6. Verfahren nach einem der Ansprüche 1 bis 4, **dadurch gekennzeichnet, dass** FAME/FAEE durch Zugabe eines anderen Alkohols unter geeigneten Reaktionsbedingungen in den entsprechenden anderen Ester umgewandelt und die Änderung der Konzentration an FAME/FAEE in der Probe anhand der Abnahme der Intensität der Carbonylbande des Esters und/oder der Zunahme der Intensität der Carbonylbande des anderen Esters gemessen wird.

7. Verfahren nach Anspruch 5 oder 6, **dadurch gekennzeichnet, dass** die Reaktion enzymatisch katalysiert ist.

8. Verfahren nach einem der Ansprüche 2 bis 7, **dadurch gekennzeichnet, dass** die Messung der Änderung der Konzentration an Analyt durch IR-Spektroskopie anhand der Abnahme der Carbonylbande bei 1749 $cm^{-1}$ bzw. 1742 $cm^{-1}$ erfolgt.

9. Verfahren nach Anspruch 8, **dadurch gekennzeichnet, dass** die Messung der Änderung der Konzentration an Analyt durch IR-Spektroskopie anhand der Abnahme der Carbonylbande bei 1749 $cm^{-1}$ durch symmetrische Auswertung um 1742 $cm^{-1}$ erfolgt bzw. anhand der Abnahme der Carbonylbande bei 1742 $cm^{-1}$ durch symmetrische Auswertung um 1749 $cm^{-1}$ erfolgt.

10. Verfahren nach einem der Ansprüche 1 bis 9, **dadurch gekennzeichnet, dass** das Verfahren zusätzlich zur quantitativen Bestimmung unter Heranziehung der/einer Reaktionskinetik eine qualitative Bestimmung aufweist.

11. Verwendung eines Verfahrens nach einem der Ansprüche 1 bis 10, **dadurch gekennzeichnet, dass** das Verfahren zur Identifizierung bzw. Ausschluss der Identität der Verunreinigung verwendet wird.

## Claims

1. A method for the quantitative determination of contaminants in the form of fatty acid esters in jet fuels, wherein the analyte is the fatty acid ester fatty acid methyl ester (FAME) and/or the fatty acid ester fatty acid ethyl ester (FAEE), wherein the analyte undergoes a chemical reaction which is selective for it and which influences the intensity for the carbonyl band of the respective ester group with the formation of a modified analyte and the variation in the concentration of analyte in the sample, which is the jet fuel together with FAME and/or FAEE, is measured using the reduction in the intensity of the carbonyl band and/or the increase in the concentration of the modified analyte is measured using the increase in the intensity of a band which is characteristic for the modification.

2. The method as claimed in claim 1, **characterized in that** the measurement of the variation in the concentration of analyte is carried out using IR spectroscopy.

3. The method as claimed in claim 2, **characterized in that** the measurement of the variation in the concentration of analyte is carried out using FTIR spectroscopy.

4. The method as claimed in claim 2, **characterized in that** the measurement of the variation in the concentration of analyte is carried out using quantum cascade lasers.

5. The method as claimed in one of claims 1 to 4, **characterized in that** FAME/FAEE is transformed into the corresponding amide by adding an amine, wherein the variation in the concentration of FAME/FAEE in the sample is measured-with the aid of the reduction in the intensity of the carbonyl band for the ester and/or the increase in the intensity of the amide band for the amide which is formed.

6. The method as claimed in one of claims 1 to 4, **characterized in that** FAME/FAEE is transformed into the corresponding other ester by adding another alcohol under suitable reaction conditions and the variation in the concentration of FAME/FAEE in the sample is measured with the aid of the reduction in the intensity of the carbonyl band for the ester and/or the increase in the intensity of the carbonyl band of the other ester.

7. The method as claimed in claim 5 or claim 6, **characterized in that** the reaction is enzymatically catalysed.

8. The method as claimed in one of claims 2 to 7, **characterized in that** the measurement of the variation in the concentration of analyte is carried out by means of IR spectroscopy with the aid of the reduction in the carbonyl band at 1749 $cm^{-1}$ or 1742 $cm^{-1}$.

9. The method as claimed in claim 8, **characterized in that** the measurement of the variation in the concentration of analyte is carried out by means of IR spectroscopy with the aid of the reduction in the carbonyl band at 1749 $cm^{-1}$ by symmetrical evaluation about 1742 $cm^{-1}$ or with the aid of the reduction in the carbonyl band at 1742 $cm^{-1}$ by symmetrical evaluation about 1749 $cm^{-1}$.

10. The method as claimed in one of claims 1 to 9, **characterized in that** in addition to the quantitative determination, the method additionally comprises a qualitative determination using the reaction kinetics or reaction kinetics.

11. Use of a method as claimed in one of claims 1 to 10, **characterized in that** the method is used for identification of the contaminant or for the exclusion of the contaminant.

## Revendications

1. Procédé pour la détermination quantitative d'impuretés sous forme d'esters d'acides gras dans des carburants pour turbines d'aéronefs, dans lequel l'analyte des esters d'acides gras est l'ester d'acide gras ester méthylique d'acide gras (FAME) et/ou l'ester d'acide gras ester éthylique d'acide gras (FAEE), l'analyte étant soumis à une réaction

chimique sélective pour cet analyte, influençant l'intensité des bandes carbonyle du groupe ester considéré, avec formation d'un analyte modifié, et la variation de la concentration de l'analyte dans l'échantillon, qui est le carburant pour turbines d'aéronefséacteur en même temps que le mélangé au FAME et/ou au FAEE, étant mesurée en ayant recours à la diminution de l'intensité de la bande carbonyle et/ou l'augmentation de la concentration de l'analyte modifié étant mesurée en faisant appel à l'augmentation de l'intensité d'une bande caractéristique pour la modification.

**2.** Procédé selon la revendication 1, **caractérisé en ce que** la mesure de la variation de la concentration de l'analyte est effectuée par spectroscopie IR.

**3.** Procédé selon la revendication 2, **caractérisé en ce que** la mesure de la variation de la concentration de l'analyte est effectuée par spectroscopie FTIR.

**4.** Procédé selon la revendication 2, **caractérisé en ce que** la mesure de la variation de la concentration de l'analyte est effectuée par spectroscopie IR en utilisant un laser à cascade quantique.

**5.** Procédé selon l'une des revendications 1 à 4, **caractérisé en ce que** le FAME/FAEE est, par addition d'une amine, converti en l'amide correspondant, la variation de la concentration du FAME/FAEE dans l'échantillon étant mesurée à l'aide de la diminution de l'intensité de la bande carbonyle de l'ester et/ou de l'augmentation de l'intensité de la bande amide de l'amide formé.

**6.** Procédé selon l'une des revendications 1 à 4, **caractérisé en ce que** le FAME/FAEE est, par addition d'un autre alcool, dans des conditions de réaction appropriées, converti en l'autre ester correspondant, et la variation de la concentration du FAME/FAEE dans l'échantillon est mesurée à l'aide de la diminution de l'intensité de la bande carbonyle de l'ester et/ou de l'augmentation de l'intensité de la bande carbonyle de l'autre ester.

**7.** Procédé selon la revendication 5 ou 6, **caractérisé en ce que** la réaction est catalysée par voie enzymatique.

**8.** Procédé selon l'une des revendications 2 à 7, **caractérisé en ce que** la mesure de la variation de la concentration de l'analyte est effectuée par spectroscopie IR à l'aide de la diminution de la bande carbonyle à 1749 $cm^{-1}$ ou 1742 $cm^{-1}$.

**9.** Procédé selon la revendication 8, **caractérisé en ce que** la mesure de la variation de la concentration de l'analyte est effectuée par spectroscopie IR à l'aide de la diminution de la bande carbonyle à 1749 $cm^{-1}$ par évaluation symétrique autour de 1742 $cm^{-1}$, ou à l'aide de la diminution de la bande carbonyle à 1742 $cm^{-1}$ par évaluation symétrique autour de 1749 $cm^{-1}$.

**10.** Procédé selon l'une des revendications 1 à 9, **caractérisé en ce que** le procédé comprend une détermination qualitative en plus de la détermination quantitative faisant appel à la ou à une cinétique de réaction.

**11.** Utilisation d'un procédé selon l'une des revendications 1 à 10, **caractérisée en ce que** le procédé est utilisé pour identifier ou exclure l'identité de l'impureté.

Fig. 1:

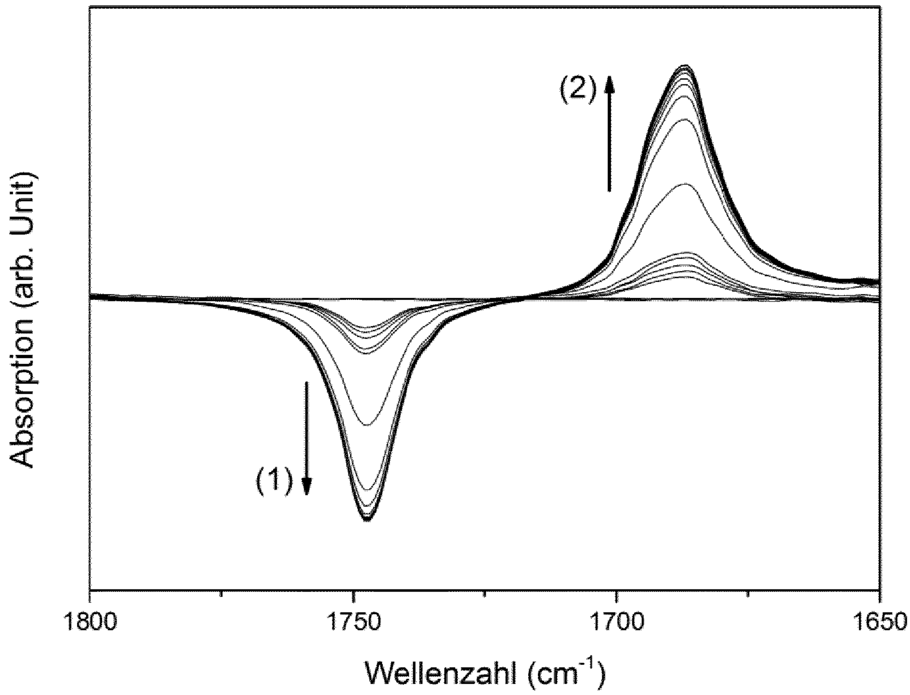
(2)
(1)
Absorption (arb. Unit)
1800
1750
1700
1650
Wellenzahl ($cm^{-1}$)

Fig. 2:

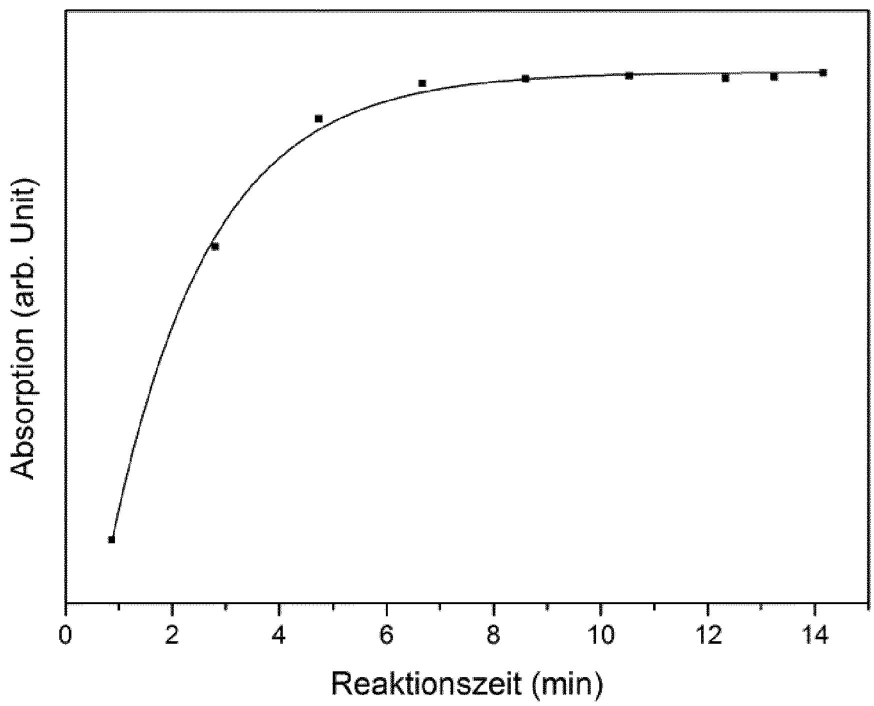
Absorption (arb. Unit)
0
2
4
6
8
10
12
14
Reaktionszeit (min)

Fig. 3:

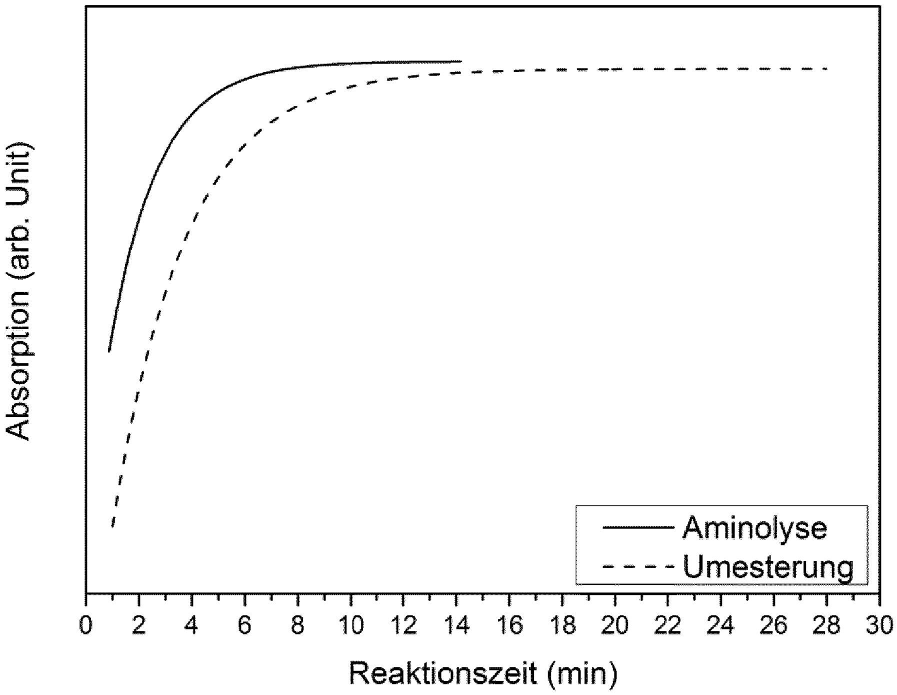
Absorption (arb. Unit)
Aminolyse
Umesterung
0 2 4 6 8 10 12 14 16 18 20 22 24 26 28 30
Reaktionszeit (min)

Fig. 4:

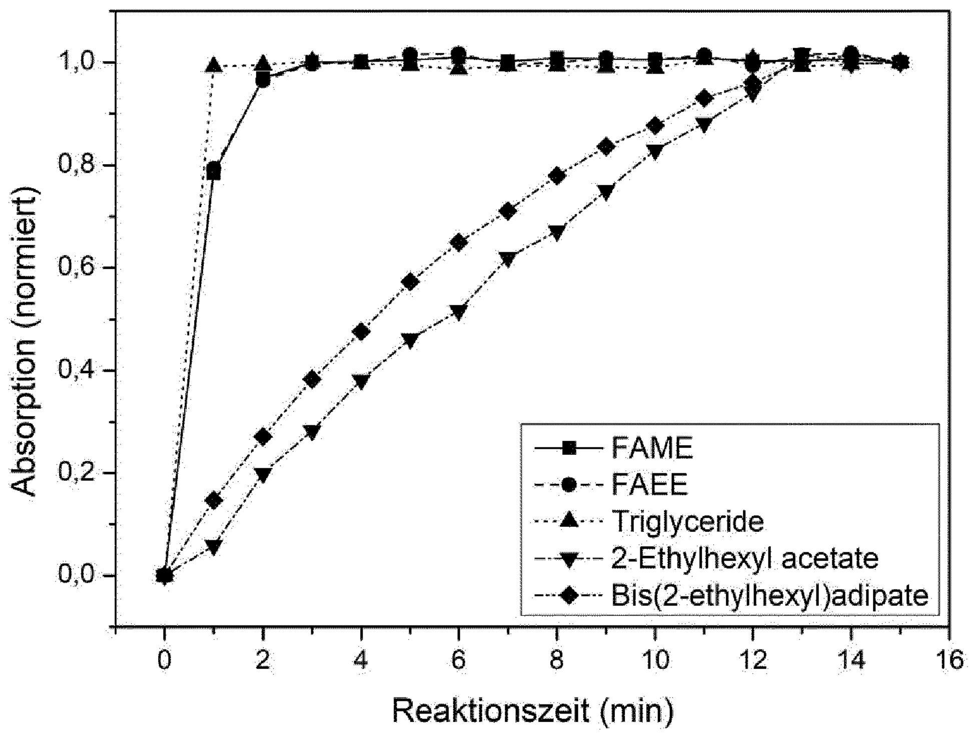
1,0
0,8
0,6
0,4
0,2
0,0
Absorption (normiert)
FAME
FAEE
Triglyceride
2-Ethylhexyl acetate
Bis(2-ethylhexyl)adipate
0 2 4 6 8 10 12 14 16
Reaktionszeit (min)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur

- **K ODEN et al.** *Analysis of Biodiesel Fatty Acid Methyl Esters (FAME) in Aviation Jet Fuels Using Method IP 585,* 2016